**Europäisches Patentamt**

**European Patent Office**

**Office européen des brevets**

(19)

(11) Veröffentlichungsnummer: **0 006 415**
**A1**

(12) # EUROPÄISCHE PATENTANMELDUNG

(21) Anmeldenummer: 78810006.3

(22) Anmeldetag: 29.06.78

(51) Int. Cl.³: **A 61 F 1/00**

---

(43) Veröffentlichungstag der Anmeldung: 09.01.80
**Patentblatt 80/1**

(84) Benannte Vertragsstaaten: CH DE FR GB

(71) Anmelder: **OSTEO AG, Bielstrasse 620, CH-2545 Selzach (CH)**

(72) Erfinder: **Mittelmeier, Heinz, Prof., An der Schutzhütte, D- Blieskastel (DE)**
Erfinder: **Moser, Heinz, Späretweg 440, CH-2545 Selzach, CH-2545 Selzach (CH)**
Erfinder: **Leu, Beat, Hintere Gasse 33, CH-2500 Biel (CH)**

(74) Vertreter: **Seehof, Michel et al, c/o AMMANN PATENTANWAELTE AG BERN Schwarztorstrasse 31, CH-3001 Bern (CH)**

---

(54) **Vorrichtung zum Einbringen von Knochenzement.**

(57) Die Vorrichtung zum Einbringen von Knochenzement in die die Prothese aufnehmende Markhöhle weist eine Applikationshülse (2) und einen Stempel (4) zum Zurückhalten des Knochenzementes beim Herausziehen der Applikationshülse auf. Durch diese Vorrichtung kann der Zement ohne Untermischung von Blut eingebracht werden. Besonders vorteilhaft ist eine solche Applikationshülse, falls der Knochenzement mittels eines Kohlefaser-Strumpfes verstärkt ist. Dabei ist es zweckmässig, den Kohlefaser-Strumpf zuerst unter Vakuum mit dem Zement zu durchtränken, wobei entweder die Applikationshülse selbst mit einem Anschluss an die Vakuumpumpe versehen sein kann oder es kann eine Röhre mit einem Anschlussstutzen für die Vakuumpumpe vorgesehen sein, in welcher der Strumpf durchtränkt wird, um anschliessend in die Applikationshülse gebracht zu werden.

EP 0 006 415 A1

- 1 -

## Vorrichtung zum Einbringen von Knochenzement

Die vorliegende Erfindung bezieht sich auf eine Vorrichtung zum Einbringen von Knochenzement ohne Untermischung von Blut in die die Prothese aufnehmende Markhöhle.

Das Einbringen des Knochenzementes in Knochenhöhlen erfolgt teils manuell durch Hineinstopfen der bereits relativ zäh gewordenen Zementmasse oder mittels Injektionsspritzen in noch flüssigem Zustand. Erstere Methode hat den Nachteil, dass es dabei zu Zwischenlagerungen von Blut mit Lamellenbildung des Zementes und damit erhöht zu Zementbrüchigkeit kommt. Die Injektion des Zementes mittels Spritzen in dünnflüssigem Zustand beinhaltet die Gefahr der toxischen Kreislauf- und Atemschädigung durch die grossen teils noch nicht abgebundenen, ins Blut übertretenden Monomere und kann Blutbeimengungen sowie Lamellenbildungen auch nicht vollständig vermeiden, da der aus der Spritze austretende Zement beim Hinunterfliessen in die Markhöhle unmittelbaren Kontakt mit der Knochenwandung hat, so dass unvermeidlich auch Turbulenzen und Blutuntermischung erfolgen. Die Folge der Blutuntermischung und Lamellenbildung ist jedoch eine ungenügende Festigkeit des Knochenzementes mit der Gefahr nachfolgender Zement- brüche und damit Prothesenlockerungen, welche infolge
der damit verbundenen Schmerzen zu Nachoperationen mit
Austausch der Prothese oder - da dies vielfach gar nicht
mehr möglich - Rückzug auf funktionsungünstigere Zustände, beispielsweise Gelenkversteifung bedingt.

Es ist daher ein Ziel der vorliegenden Erfindung, eine
Vorrichtung anzugeben, die obige Nachteile vermeidet.
Dieses Ziel wird durch eine Vorrichtung erreicht, die
dadurch gekennzeichnet ist, dass sie eine Applikationshülse in etwa der Form des einzusetzenden Prothesenteiles und einen Stempel zum Zurückhalten des Knochenzementes beim Herausziehen der Applikationshülse aus
der Markhöhle aufweist. Bei einem bevorzugten Ausführungsbeispiel besteht die Applikationshülse aus durchsichtigem
Kunststoff und ist schlitzbar, während der Stempel in
einer weiteren vorzugsweisen Ausführung ebenfalls aus
Kunststoff besteht und mit einem Haltegriff versehen ist.

Die Erfindung wird nachfolgend anhand einer Zeichnung eines Ausführungsbeispiels näher erläutert werden. Es
zeigen:

     Figur 1: einen Kohlefaser-Strumpf,

     Figur 2: eine Vorrichtung zum Einbringen des Knochenzementes mit dem Faserstrumpf,

     Figur 3: einen Ausschnitt aus Figur 2 und

     Figur 4: eine Applikationshülse.

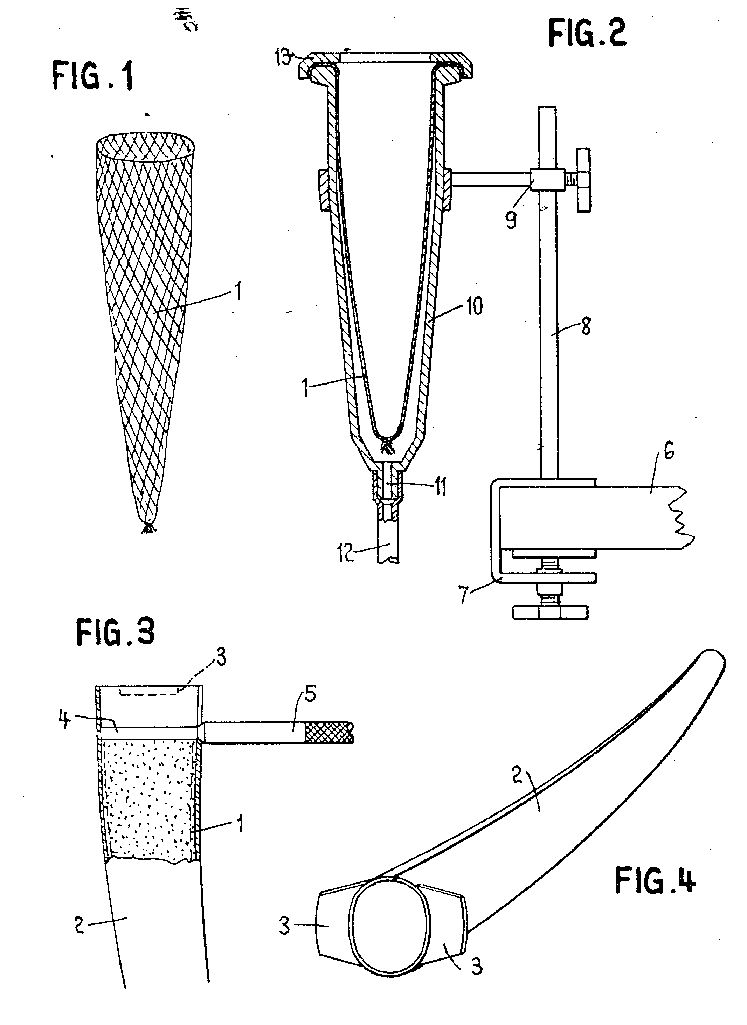

Die Vorrichtung zum Einbringen des Knochenzementes enthält eine Applikationshülse 2 mit Haltebacken 3 (Figur 4),
vorzugsweise aus flexiblem, durchsichtigem Kunststoff, bei-

0006415

spielsweise Polyäthylen. Die Zementapplikation ohne Untermischung mit Blut wird dadurch erreicht, dass der Zement in noch relativ dünnflüssigem Zustand in die Applikationshülse eingebracht wird, die eine zylindrische, konische, im Querschnitt eventuell ovale und, im Falle einer Hüftgelenkprothese, der Länge nach gekrümmte Form aufweist und dann mit der Hülse in die entsprechend vorbereitete Markhöhle so tief als möglich eingesteckt wird. Die Hülse verhindert dabei Turbulenzen der Zementflüssigkeit und Durchmischung des Zementes mit dem Blut an den Wänden der Knochenhöhle. Sodann wird an der bestehenden Oeffnung ein Stempel 4 in die Hülse eingesetzt und anschliessend die Hülse über dem Stempel zurückgezogen, so dass der Zement mittels des Stempels in der Marhöhle gehalten wird. Erst durch das Zurückziehen der Hülse erlangt der Zement Kontakt mit dem blutigen, umgebenden Knochengewebe. Durch das darauffolgende Einsetzen des Prothesenstieles, welcher Volumen verdrängt, wird der Knochenzement dann fest gegen den Knochen gepresst und die Verzahnung der Zementoberfläche mit dem Knochen herbeigeführt. Dabei wird die Möglichkeit, vor dem Einsetzen des Zementes, wie vorbekannt, ein dünnes Plastikschläuchlein in die Tiefe der Markhöhle einzuführen, um das Entweichen von Luft und Blut aus der Tiefe der Markhöhle während der Zementeinbringung zu ermöglichen, beibehalten; wobei dieses Schläuchlein bereits an der Applikationshülse befestigt sein kann und diese in dieser Form geliefert werden kann. Um bei konischer Form der Markhöhle und bei ovalem Querschnitt der Hülse letztere über den Stempel ohne Schwierigkeiten zurückziehen zu können, sollte die Hülse mit sich überlappenden Rändern geschlitzt werden, so dass sich das unten enger werdende Hülsenrohr durch Aufweiten des Schlitzes über dem Haltestempel zurückziehen lässt. Dadurch lässt sich beim Einführen derselben ein tieferes Eindringen ermöglichen. Es ist selbstverständlich auch möglich, die Applikationshülse aus einer dünnen, flexiblen Metallfolie auszuführen.

Besonders vorteilhaft ist die Verwendung der Applikationshülse für das Einbringen von faserverstärktem Zement. Dabei wird in erster Linie an ein Zement mit einer Verstärkung in Form eines Kohlefaser-Strumpfes 1 gedacht, wie er
in Figur 1 skizziert ist. Dabei kann so vorgegangen werden,
dass dieser Kohlefaser-Strumpf in die Applikationshülse
gesteckt und mit frisch angerührtem, flüssigem Knochenzement ausgegossen wird, um sodann in die Knochenhöhle
eingebracht zu werden. Damit wird zugleich verhütet, dass
der Faserstrumpf beim Einbringen an der inneren Rauhigkeit
des Knochens aufgerissen oder festgehalten wird. Es ist
auch hier möglich, dem Prothesenstiel in den den Faserstrumpf ausfüllenden Knochenzement schon vor dem Einbringen desselben in den Knochen einzustossen und gemeinsam
mit dem Faserschlauch und Kunststoffzement in den Knochen
einzuführen.

Zur Erleichterung der Zementfüllung und insbesondere zur
luftfreien Durchtränkung des Faserschlauches mit Knochenzement ist die Zusatzeinrichtung gemäss Figur 2 von grossem
Nutzen. Man erkennt dort den mit 6 angedeuteten Operationstisch, den mittels einer Klemmhalterung 7 daran befestigten
Haltestab 8, an welchem eine auf diesem Stab gleitende
Halterung 9 befestigbar ist. Diese Halterung trägt eine
Kunststoffröhre 10, die eine ähnliche Form wie die vorgehend beschriebene Applikationshülse aufweist, d. h. einen
zylindrischen, oder konischen und/oder ovalen Querschnitt.
Diese Kunststoffröhre 10 weist am unteren Ende einen Anschlussstutzen 11 für einen Evakuierungsschlauch 12, der
an eine nicht eingezeichnete Vakuumpumpe angeschlossen
werden kann, auf. Das freie Ende des Faserstrumpfes 1 ist
über den oberen Rand der Kunststoffröhre umgeschlagen und
dort mit einem klemmenden Ueberwurfring 13 aus Kunststoff

0006415

befestigt, wobei dieser Ring geschlitzt sein kann. Nach dem Anschluss des Schlauches 12 an die Vakuumpumpe wird der frisch angerührte dünnflüssige Kunststoffzement, gegebenenfalls mit Apatitbeimengung für das bessere Anwachsen des Knochens von oben in den in der Kunststoffröhre hängenden Faserstrumpf eingegossen. Infolge des Soges wird der Kunststoff fest in die Strumpfmaschen hineingesogen, so dass das Maschenwerk völlig durchtränkt und an der Aussenseite des Faserschlauches sogar mit kleinen Protusionen vorsteht. Sobald der Zement eine zähflüssige Konsistenz angenommen hat, wird der zementgefüllte Faserschlauch aus dem Füllgefäss herausgenommen und in die Applikationshülse 2 eingelegt. Dies kann am besten dadurch erfolgen, dass die Applikationshülse am Schlitz zeitweise aufgebogen wird und nach Einlegen des mit Zement gefüllten Faserstrumpfes wieder zusammengeschnappt, bzw. zusammengedrückt wird. Nachher kann die Applikationshülse mit dem faserverstärkten Zement in die Markhöhle eingebracht werden und anschliessend die Hülse hinausgezogen werden, wobei der Zement mit der Faserverstärkung durch den Stempel 4, der am Haltegriff 5 gehalten werden kann, an Ort und Stelle gehalten wird. Anschliessend wird der Prothesenverankerungsstiel hineingetrieben.

Es ist aber auch möglich, den Prothesenverankerungsstiel bereits in den zementgefüllten Faserstrumpf einzuführen, solange derselbe noch im Evakuierungsgefäss steckt. Es ist auch möglich, den Faserstrumpf mit der geschlitzten Applikationshülse in das Evakuierungsgefäss einzusetzen.

Im Zuge einer weiteren Vereinfachung ist es möglich, die Applikationshülse selbst als Evakuierungsgefäss auszubilden, indem sie zunächst als geschlossenes, jedoch leicht aufschlitzbares Rohr und an der Spitze mit einem Anschlussstutzen für den Evakuierungsschlauch gefertigt wird. Nach

0006415

erfolgter Einfüllung des Zementes wird dann der Anschlussstutzen mit der Schere abgeschnitten und die Hülse beispielsweise mit der Schere aufgeschlitzt, um sie dann als Applikationshülse zu benutzen. Es ist aber auch möglich, das Aufschlitzen der Applikationshülse durch einen eingeklebten Faden oder dergleichen zu ermöglichen. Um das Befestigen des Faserstrumpfes zu erleichtern, kann dieser bereits mit einem daran befestigten Ueberwurfring versehen sein. Ausserdem kann das Einführen des zementgefüllten Faserstrumpfes in die Applikationshülse durch eine Einrichtung zum Aufspreizen derselben erleichtert werden.

Es ist vorteilhaft, den Stempel 4 aus wegwerfbarem Kunststoffmaterial anzufertigen, während der Haltegriff 5, am vorteilhaftesten senkrecht zur Hülsenoberfläche am Stempel befestigt, als Schlitzöffner dient und entsprechend geformt sein kann. Es ist aber auch vorgesehen, den Haltegriff senkrecht auf den Stempel zu befestigen oder ihn ge-krümmt auszuführen.

- 1 -

Patentansprüche:

1. Vorrichtung zum Einbringen von Knochenzement ohne Untermischung von Blut in die die Prothese aufnehmende Markhöhle,
   dadurch gekennzeichnet,
   dass sie eine Applikationshülse (2) in etwa der Form des einzusetzenden Prothesenteiles und einen Stempel (4) zum Zurückhalten des Knochenzementes beim Herausziehen der Applikationshülse aus der Markhöhle, aufweist.

2. Vorrichtung gemäss Anspruch 1,
   dadurch gekennzeichnet,
   dass die Applikationshülse aus durchsichtigem Kunststoff besteht.

3. Vorrichtung gemäss Anspruch 1,
   dadurch gekennzeichnet,
   dass die Applikationshülse aus einer Metallfolie besteht.

4. Vorrichtung gemäss einem der Ansprüche 1 bis 3,
   dadurch gekennzeichnet,
   dass die Applikationshülse aufschlitzbar ist und sich überlappende Ränder aufweist.

0006415

5. Vorrichtung gemäss einem der Ansprüche 1 bis 4,
dadurch gekennzeichnet,
dass die Applikationshülse (2) an ihrem verjüngten
Ende einen Anschlussstutzen zum Anschluss an eine Vakummpumpe aufweist.

6. Vorrichtung gemäss einem der Ansprüche 1 bis 4,
dadurch gekennzeichnet,
dass sie ferner eine der Form der Applikationshülse
entsprechende beidseitig geöffnete Röhre (10) mit einem Anschlussstutzen (11) für einen Evakuierungsschlauch
(12) an ihrem verjüngten Ende und eine Ueberwurfmutter
(13) zum befestigen eines Kohlefaser-Strumpfes (1) an
ihrem anderen Ende aufweist und dass diese Röhre durch
eine am Operationstisch befestigbare Halterung (9) gehalten ist.

7. Vorrichtung gemäss Anspruch 1,
dadurch gekennzeichnet,
dass der zum einmaligen Gebrauch bestimmte Stempel (4)
aus Kunststoff einen in der Ebene des Stempels angebrachten Haltegriff (5) aufweist.

8. Vorrichtung gemäss Anspruch 7,
dadurch gekennzeichnet,
dass der Haltegriff (5) Mittel zum Auftrennen der
Applikationshülse (2) aufweist.

9. Vorrichtung gemäss einem der Ansprüche 1 bis 7,
dadurch gekennzeichnet,
dass die Applikationshülse einen eingelegten Faden zum
Aufreissen der Hülse aufweist.

10. Vorrichtung gemäss einem der Ansprüche 1 bis 9,
dadurch gekennzeichnet,

- 3 -

0006415

dass die Applikationshülse Haltebacken (3) aufweist.

11. Vorrichtung gemäss einem der Ansprüche 1 bis 10,
dadurch gekennzeichnet,
dass die Applikationshülse einen daran befestigten, dem
Entweichen von Blut und Luft dienenden Kunststoffschlauch
aufweist.

0006415

FIG.1

FIG.2

FIG.3

FIG.4

## EUROPÄISCHER RECHERCHENBERICHT

Europäisches Patentamt

0006415

Nummer der Anmeldung

EP 78 81 0006

| Kategorie | Kennzeichnung des Dokuments mit Angabe, soweit erforderlich, der maßgeblichen Teile | betrifft Anspruch | KLASSIFIKATION DER ANMELDUNG (Int.Cl.²) |
|---|---|---|---|
| | **EINSCHLÄGIGE DOKUMENTE** | | |
| | DE - B - 2 334 643 (FRIEDRICHS-FELD) <br><br> * Spalte 1, Zeilen 4-20 (Patentansprüche 1-4);Spalte 2, Zeilen 49-59 * | 1,2,3 | A 61 F 1/00 |
| | US - A - 4 064 567 (BURSTEIN und KOSLIN) <br><br> * Abstract; Spalte 3, Zeilen 60-66; Spalte 4, Zeilen 55-65 * | 1,2,3 | |
| | DE - A - 2 621 666 (REIMER und LYSELL) <br><br> * Seite 11, Zeilen 1-18 * | 5,11 | RECHERCHIERTE SACHGEBIETE (Int Cl.³) <br><br> A 61 F <br> A 61 B |

Der vorliegende Recherchenbericht wurde für alle Patentansprüche erstellt.

| Recherchenort | Abschlußdatum der Recherche | Prüfer |
|---|---|---|
| Den Haag | 17-04-1979 | PESCHEK |

EPA form 1503.1 06.78